# EUROPEAN PATENT APPLICATION

(11) **EP 4 560 017 A1**
(43) Date of publication of application: **28.05.2025**
(21) Application number: 23843051.6
(22) Date of filing: 20.07.2023
(51) Int. Cl.: C12N 9/42, C12N 9/24, C12N 9/26, C12N 9/88, C12N 15/56, C12N 15/60, C13K 1/02

(54) **PLANT BIOMASS SACCHARIFYING ENZYME COMPOSITION**

(30) Priority: 20.07.2022 JP 2022115849
(71) Applicant: Kao Corporation, Tokyo 103-8210 (JP)
(72) Inventor: LIN, Meng-i, Wakayama-shi, Wakayama 640-8580 (JP); ICHINOSE, Sakurako, Wakayama-shi, Wakayama 640-8580 (JP); SHIBATA, Nozomu, Wakayama-shi, Wakayama 640-8580 (JP); TAKAHASHI, Fumikazu, Wakayama-shi, Wakayama 640-8580 (JP)
(74) Representative: Vossius & Partner Patentanwälte Rechtsanwälte mbB
(86) International application number: PCT/JP2023/026684
(87) International publication number: WO 2024/019128

(57) **Abstract**

Provided are an enzyme preparation which is effective for viscosity reduction in the saccharification treatment of plant biomass, and a method for producing the same. An enzyme composition comprising the following (A), (B), and (C): (A) cellulase; (B) a homogalacturonan-degrading enzyme containing polygalacturonase and pectin lyase; and (C) one or more members selected from the group consisting of arabinofuranosidase and galactanase.

## Description

### Field of the Invention

The present invention relates to a plant biomass saccharifying enzyme composition.

### Background of the Invention

Biorefinery technology, which produces sugar and value-added products from renewable plant biomass raw materials, is expected to be a way to build a sustainable society. Among biomass raw materials, agricultural wastes containing starch and cellulose can be degraded to obtain a large amount of glucose, which is the most commonly used sugar as a fermentation raw material. Therefore, many technological developments have been carried out to recover sugar from these biomass raw materials. Plant biomass raw materials are known to also contain hemicellulose, pectin, and lignin, in addition to starch and cellulose.

Pectin is a complex polysaccharide which is predominantly present in primary cell walls of plants and in the middle lamella between cells, and which is mainly composed of three domains, i.e., homogalacturonan (HG), rhamnogalacturonan I (RG-I), and rhamnogalacturonan II (RG-II). HG is a polymer mainly composed of polygalacturonic acid which is α-1,4-linked with D-galacturonic acid modified by methyl esterification of a part of the carboxyl group at position 6 and acetylation of a part of the hydroxyl groups at positions 2 and 3. Rhamnogalacturonan I (RG-I) is a polymer which has a disaccharide repeating structure of rhamnose and galacturonic acid as the main chain, and which has a galactan side chain composed of a series of galactose residues, an arabinan side chain composed of a series of arabinose residues, and an arabinogalactan side chain composed of arabinose and galactose residues. As with HG, rhamnogalacturonan II (RG-II) has α-1,4-linked polygalacturonic acid as the main chain, and is the most structurally complex domain of pectin, in which side chains including about 30 types of sugars, such as L-rhamnose, D- or L-galactose, L-arabinose, D-galacturonic acid, D-glucuronic acid, D-apiose, L-fucose, 2-O-methyl-L-fucose, 2-O-methyl-D-xylose, aceric acid (3-O-carboxy-5-deoxy-L-xylose), 2-keto-3-deoxy-D-manno-2-octuronic acid, and 3-deoxy-D-lyxo-2-hepturonic acid, are linked to the O-2 or O-3 positions of galacturonic acid.

The constituents of pectin do not contain glucose, and the amount of glucose which can be recovered from plant residues is not considered to be affected; however, the high viscosity of pectin poses a major challenge to the efficiency of sugar recovery from sugar-containing liquid obtained from the degradation of plant biomass and to the sugar production process.

The saccharification process of pectin-containing plant biomass using enzymes requires amylase and amyloglucosidase, which convert starch to glucose, and cellulase, which converts cellulose to glucose. In addition to these, cellulase is used in combination with pectinase in order to reduce the viscosity of sugar-containing liquid. For example, addition of polygalacturonase (Patent Literature 1), and addition of arabinan endo-1,5-α-L-arabinosidase (Patent Literature 2) are known.

However, their viscosity reduction effects are not always sufficient. In addition, commercially available pectinase preparations are not practical in terms of cost because they contain various types of enzymes other than those involved in viscosity reduction, resulting in large amounts of enzymes required.

Therefore, for the saccharification of pectin-containing plant biomass, there is a need for enzyme preparations which can reduce the viscosity of sugar-containing liquid at low cost.

[Patent Literature 1] JP-B-59-37953
[Patent Literature 2] WO 2015/097017

### Summary of the Invention

The present invention relates to the followings 1) to 9).
1) An enzyme composition comprising the following (A), (B), and (C):
   (A) cellulase;
   (B) a homogalacturonan-degrading enzyme containing polygalacturonase and pectin lyase; and
   (C) one or more members selected from the group consisting of arabinofuranosidase and galactanase.
2) A method for saccharifying plant biomass, comprising bringing the enzyme composition of 1) into contact with plant biomass.
3) A method for producing sugar from plant biomass, comprising bringing the enzyme composition of 1) into contact with plant biomass.
4) A method for producing ethanol from plant biomass, comprising bringing the enzyme composition of 1) into contact with plant biomass.
5) A recombinant filamentous fungus comprising (b1) a polygalacturonase gene, (b2) a pectin lyase gene, and (c) one or more genes selected from the group consisting of arabinofuranosidase gene and galactanase gene which are introduced thereinto.
6) A method for producing the enzyme composition of 1), comprising culturing the recombinant filamentous fungus of 5).
7) An agent for reducing a viscosity of plant biomass comprising the enzyme composition of 1) as an active ingredient.
8) A method for reducing a viscosity of plant biomass, comprising bringing the enzyme composition of 1) into contact with plant biomass.
9) Use of the enzyme composition of 1) for reducing plant biomass viscosity.

### Detailed Description of the Invention

The present invention relates to the provision of an enzyme preparation which is effective for viscosity reduction in the saccharification treatment of plant biomass, and a method for producing the same.

The present inventors found that an enzyme composition containing cellulase and a specific pectinase can effectively suppress the increase in the viscosity of slurry when pectin-containing plant biomass is saccharified, and that the enzyme composition can be easily produced by using a recombinant filamentous fungus.

The present invention makes it possible to provide at low cost a saccharifying enzyme composition which can suppress the increase in the viscosity of slurry in the saccharification treatment process of pectin-containing plant biomass, and which allows efficient saccharification treatment of plant biomass.

### (1. Enzyme Composition)

The enzyme composition of the present invention contains the following (A), (B), and (C):
(A) cellulase;
(B) a homogalacturonan-degrading enzyme containing polygalacturonase and pectin lyase; and
(C) one or more members selected from the group consisting of arabinofuranosidase and galactanase.

The cellulase (A) is a generic name for enzymes which degrade cellulose, and includes endoglucanases which cleave a cellulose molecule internally (EC 3.2.1.4), exoglucanases which degrade cellulose at the reducing end or non-reducing end to liberate cellobiose (cellobiohydrolase, EC 3.2.1.91), and β-glucosidase (EC 3.2.1.21); however, in the present invention, the cellulase may belong to any of these classes, and several types can be used in combination.

As the origin of the cellulase, *Bacillus subtilis, Aspergillus niger, Irpex lacteus, Humicura insorens, Trichoderma viride, Trichoderma reesei,* and the like are known. In the present invention, it is preferable to use cellulase derived from the genus *Trichoderma.*

In the present invention, the cellulase can be in the form of a culture obtained by solid culture or liquid culture of the above cellulase-producing microorganisms in accordance with a general method, a concentrated product obtained by purifying it, or a crude enzyme powder obtained by drying these. Other usable examples include commercially available cellulase preparations, such as Celluclast (NOVOZYME), Meicelase (Meiji Seika Pharma Co., Ltd.), and Sumizyme C and Sumizyme AC (Shinnihon Chemicals Corporation).

In terms of the saccharification of biomass and the suppression of the increase in the viscosity of slurry, the cellulase is blended so that, for example, the cellulase activity of the enzyme composition is 2.0 U or more, preferably 2.2 U or more, and more preferably 2.4 U or more, and is 5.0 U or less, preferably 4.0 U or less, and more preferably 3.6 U or less, or is from 2.0 to 5.0 U, preferably from 2.2 to 4.0 U, and more preferably from 2.4 to 3.6 U, per mg of protein in the enzyme composition.

The cellulase activity as mentioned herein is a value measured in such a manner that cellulase is reacted at a pH of 4.8 and 50°C using filter paper as a substrate, and reducing sugar, which is an enzyme reaction product, is quantified by a colorimetric method. One unit of enzyme (1 U) means the amount of enzyme which produces 1 µmol of reducing sugar equivalent to glucose per minute.

The homogalacturonan-degrading enzyme (B) is an enzyme which degrades the homogalacturonan (HG) domain in pectin, and contains at least polygalacturonase and pectin lyase in the present invention.

The polygalacturonase is an enzyme which hydrolyzes the α-1,4-glycosidic bond of D-galacturonic acid, which constitutes pectic acid (EC 3.2.1.15).

The pectin lyase is an enzyme which catalyzes the reaction to convert pectin to an oligosaccharide with unsaturated bonds between the C-4 and C-5 terminal residues (EC 4.2.2.10).

As the polygalacturonase and pectin lyase of the present invention, those derived from the genera *Aspergillus, Penicillium, Rhizopus,* and the like can be used. The polygalacturonase and pectin lyase each can be in the form of a culture obtained by solid culture or liquid culture of microorganisms with the ability to produce polygalacturonase or pectin lyase in accordance with a general method, a concentrated product obtained by purifying it, or a crude enzyme powder obtained by drying these. It is also possible to use commercially available polygalacturonase preparations and pectin lyase preparations, such as Sumizyme AP2 (Shinnihon Chemicals Corporation), Suclase N (Mitsubishi Chemical Corporation), and a polygalacturonase solution from *Aspergillus niger* (Sigma, P4716).

The polygalacturonase is preferably blended so that, for example, the polygalacturonase activity of the enzyme composition is 30 U or more per mg of protein in the enzyme composition, and is specifically 30 U or more, preferably 60 U or more, more preferably 90 U or more, and more preferably 150 U or more, and is 1 200 U or less, preferably 600 U or less, and more preferably 450 U or less, or is preferably from 30 to 1 200 U, more preferably from 60 to 600 U, more preferably from 90 to 450 U, and more preferably from 150 to 450 U.

The polygalacturonase activity as mentioned herein is a value measured in such a manner that polygalacturonase is reacted at a pH of 4.0 and 50°C using a polygalacturonic acid solution as a substrate, and reducing sugar, which is an enzymatic reaction product, is quantified by a colorimetric method. One unit of enzyme (1 U) means the amount of enzyme which produces 1 µmol of reducing sugar equivalent to D-galacturonic acid per minute.

The pectin lyase is preferably blended so that, for example, the pectin lyase activity of the enzyme composition is 1 U or more per mg of protein in the enzyme composition, and is specifically 1 U or more, preferably 1.5 U or more, and more preferably 1.7 U or more, and is 27 U or less, preferably 16 U or less, and more preferably 10 U or less, or is preferably from 1 to 27 U, more preferably from 1.5 to 16 U, and more preferably from 1.7 to 10 U.

The pectin lyase activity as mentioned herein is a value measured in such a manner that pectin lyase is reacted at a pH of 4.0 and 50°C using a polygalacturonic acid solution as a substrate, and unsaturated oligogalacturonide, which is an enzymatic reaction product, is measured using the molar molecular absorption coefficient of unsaturated digalacturonide, i.e., 4 600 M⁻¹cm⁻¹. One unit of enzyme (1 U) means the amount of enzyme which produces 1 µmol of unsaturated oligogalacturonide per minute.

The arabinofuranosidase (c) is an enzyme which hydrolyzes the arabinose side chain at the non-reducing terminal side, and is classified as EC 3.2.1.55.

The galactanase is a hydrolase which hydrolyzes the glycosidic bond of galactan, and is classified as EC 3.2.1.89, EC 3.2.1.164, and EC 3.2.1.181.

Rhamnogalacturonan I (RG-I) in pectin is a polymer with a disaccharide repeating structure of rhamnose and galacturonic acid as the main chain, a galactan side chain composed of a series of galactose residues, an arabinan side chain composed of a series of arabinose residues, and an arabinogalactan side chain composed of arabinose and galactose residues. The arabinofuranosidase and galactanase contribute to the degradation of side chains of the rhamnogalacturonan I (RG-I) structure in pectin. From this meaning, in the present invention, the arabinofuranosidase and galactanase are also referred to as pectinases.

As the arabinofuranosidase and galactanase of the present invention, those derived from the genera *Aspergillus, Penicillium, Rhizopus,* and the like can be used. The arabinofuranosidase and galactanase can be in the form of a culture obtained by solid culture or liquid culture of microorganisms with the ability to produce arabinofuranosidase or galactanase in accordance with a general method, a concentrated product obtained by purifying it, or a crude enzyme powder obtained by drying these. Other usable examples include commercially available arabinofuranosidase preparations and galactanase preparations, such as α-L-arabinofuranosidase (*Aspergillus niger*) (Megazyme, E-AFASE), α-L-arabinofuranosidase (*Aspergillus nidulans*) (Megazyme, E-ABFAN), endo-β-1,4-galactanase (*Aspergillus niger*) (Megazyme, E-EGALN), endo-β-1,4-galactanase (*Cellvibrio japonicus*) (Megazyme, E-GALCJ), and endo-β-1,4-galactanase (*Clostridium thermocellum*) (Megazyme, E-GALCT).

The arabinofuranosidase is preferably blended so that, for example, the arabinofuranosidase activity of the enzyme composition is 0.1 U or more per mg of protein in the enzyme composition, and is specifically 0.1 U or more, preferably 1 U or more, and more preferably 3 U or more, and is 50 U or less, preferably 25 U or less, and more preferably 15 U or less, or is preferably from 0.1 to 50 U, more preferably from 1 to 25 U, and more preferably from 3 to 15 U.

The arabinofuranosidase activity as mentioned herein is a value measured in such a manner that arabinofuranosidase is reacted at a pH of 4.0 and 50°C using a 4-nitrophenyl-α-L-arabinofuranoside solution as a substrate, and 4-nitrophenol, which is an enzymatic reaction product, is quantified by a colorimetric method. One unit of enzyme (1 U) means the amount of enzyme which produces 1 µmol of 4-nitrophenol per minute.

The galactanase is preferably blended so that, for example, the galactanase activity of the enzyme composition is 3 U or more per mg of protein in the enzyme composition, and is specifically 3 U or more, preferably 5 U or more, and more preferably 20 U or more, and is 250 U or less, preferably 100 U or less, and more preferably 80 U or less, or is preferably from 3 to 250 U, more preferably from 5 to 100 U, and more preferably from 20 to 80 U.

The galactanase activity as mentioned herein is a value measured in such a manner that galactanase is reacted at a pH of 4.0 and 50°C using a galactan solution as a substrate, and reducing sugar, which is an enzymatic reaction product, is quantified by a colorimetric method. One unit of enzyme (1 U) means the amount of enzyme which produces 1 µmol of reducing sugar equivalent to galactose per minute.

The enzyme composition of the present invention can be formulated by mixing the three types of enzymes (A), (B), and (C) described above, and optionally various carriers, excipients, and other additives conventionally used for this type of enzyme preparations. The dosage form may be tablets, powders, granules, liquids, or the like, and can be formulated by a general method.

In the enzyme composition of the present invention, the content ratio of the homogalacturonan-degrading enzyme (B) and one or more members selected from the group consisting of arabinofuranosidase and galactanase (C) ((B)+(C)) contained in protein in the enzyme composition is preferably 1 mass% or more, more preferably 5 mass% or more, and more preferably 10 mass% or more, and is preferably 50 mass% or less, more preferably 45 mass% or less, and more preferably 40 mass% or less. The content ratio is also preferably from 1 mass% to 50 mass%, more preferably from 5 mass% to 45 mass%, and more preferably from 10 mass% to 40 mass%.

In the homogalacturonan-degrading enzyme (B), the usage ratio of polygalacturonase and pectin lyase is not particularly limited, and they can be mixed in any ratio.

The enzyme composition of the present invention may also contain, as appropriate, enzymes other than (A) to (C), such as amylase, glucoamylase, β-glucosidase, glucanase, hemicellulase, xylanase, xylosidase, protease, and lipase.

The enzyme composition of the present invention is an enzyme composition for saccharifying plant biomass, preferably pectin-containing plant biomass (plant biomass saccharifying agent), and more specifically an enzyme composition which can reduce the viscosity of saccharified plant biomass compared to the case of saccharification using cellulase alone.

Examples of the biomass to be treated with the enzyme composition include cellulose- and pectin-containing substances, such as cassava, sweet potatoes, potatoes, and other potato crops or their residues, corn, corn hull, apple residue, and citrus peel; preferred is cassava or cassava residue.

### (2. Production of Enzyme Composition Using Recombinant Filamentous Fungus)

The enzyme composition of the present invention can also be produced by culturing a recombinant filamentous fungus into which (b1) a polygalacturonase gene, (b2) a pectin lyase gene, and (c) one or more genes selected from the group consisting of arabinofuranosidase gene and galactanase gene are introduced.

Examples of the polygalacturonase gene (b1) include PgaB gene, PgaI gene, PgaII gene, and the like.

Such a polygalacturonase gene is preferably derived from a filamentous fungus, and preferred examples include those derived from microorganisms of the genus *Aspergillus* (e.g., *Aspergillus niger, Aspergillus japonicus,* and *Aspergillus aculeatus*)*,* microorganisms of the genus *Rhizopus* (e.g., *Rhizopus oryzae* and *Rhizopus delemar*)*,* microorganisms of the genus *Talaromyces* (e.g., *Talaromyces cellulolyticus*), microorganisms of the genus *Penicillium* (e.g., *Penicillium roqueforti*), and the like.

More preferred among these are PgaB gene of *Aspergillus niger,* PgaI gene of *Aspergillus niger,* PgaII gene of *Aspergillus niger.*

The PgaB gene of *Aspergillus niger* is registered as accession number XM_025597756 in the NCBI public database (SEQ ID NO: 1).

The PgaI gene of *Aspergillus niger* is registered as accession number XM_025596768 in the NCBI public database (SEQ ID NO: 3).

The PgaII gene of *Aspergillus niger* is registered as accession number XM_025602343.1 in the NCBI public database (SEQ ID NO: 5).

Examples of the pectin lyase gene (b2) include PelD gene, PelF gene, and the like.

Such a pectin lyase gene is preferably derived from a filamentous fungus, and examples include those derived from microorganisms of the genus *Aspergillus* (e.g., *Aspergillus niger, Aspergillus japonicus,* and *Aspergillus ludhuensis,* and *Aspergillus aculeatus*), microorganisms of the genus *Penicillium* (e.g., *Penicillium digitatum* and *Penicillium solitum*), microorganisms of the genus *Talaromyces* (e.g., *Talaromyces rugulosus* and *Talaromyces cellulolyticus*), and the like.

Preferred among these is PelD gene of *Aspergillus niger* or PelF gene of *Aspergillus niger.*

The PelD gene of *Aspergillus niger* is registered as accession number XM_025604039.1 in the NCBI database (SEQ ID NO: 7).

The PelF gene of *Aspergillus niger* is registered as accession number: XM_001401024.2 in the NCBI database (SEQ ID NO: 9).

Examples of the arabinofuranosidase gene (c) include Abf gene, and examples of the galactanase gene include Gal gene.

Such an arabinofuranosidase gene and a galactanase gene are preferably derived from filamentous fungi, and preferred examples include those derived from microorganisms of the genus *Aspergillus* (e.g., *Aspergillus niger, Aspergillus japonicus,* and *Aspergillus aculeatus*), microorganisms of the genus *Rhizopus* (e.g., *Rhizopus oryzae* and *Rhizopus delemar*), microorganisms of the genus *Talaromyces* (e.g., *Talaromyces cellulolyticus*), microorganisms of the genus *Trichoderma* (e.g., *Trichoderma reesei*), microorganisms of the genus *Penicillium* (e.g., *Penicillium roqueforti* and *Penicillium chrysogenum*), and the like.

More preferred among these are Abf gene of *Aspergillus niger* and Gal gene of *Aspergillus niger.*

The Abf gene of *Aspergillus niger* is registered as accession number L29005.1 (amino acid sequence AAC41644.1) in the NCBI database (SEQ ID NO: 11). The Gal gene of *Aspergillus niger* is registered as accession number AJ305303.1 (amino acid sequence CAC83735.1) in the NCBI database (SEQ ID NO: 13).

As the means for introducing the polygalacturonase gene (b1), pectin lyase gene (b2), and one or more genes selected from the group consisting of arabinofuranosidase gene and galactanase gene (c) of the present invention into a filamentous fungus, for example, a vector or DNA fragment containing a polynucleotide encoding each enzyme gene may be introduced into a host filamentous fungus (parent strain).

Here, the vector containing a polynucleotide encoding each enzyme gene is an expression vector, and preferably an expression vector which can introduce the polynucleotide into the host filamentous fungus and express the polynucleotide within the host. Moreover, the vector preferably contains the polynucleotide and a control region operably linked thereto. The vector may be a vector capable of autonomous growth and replication outside the chromosome, such as plasmids, or may be a vector integrated into the chromosome.

The "operable linkage" as mentioned herein means that the gene and the control region are linked such that the gene can be expressed under the control of the control region. The procedure of the "operable linkage" between the gene and the control region is well known to a person skilled in the art.

Specific examples of vectors include pBluescript II SK(-) (Stratagene), pUC-based vectors such as pUC18/19 and pUC118/119 (Takara Bio Inc.), pET-based vectors (Takara Bio Inc.), pGEX-based vectors (GE HealthCare), pCold-based vectors (Takara Bio Inc.), pHY300PLK (Takara Bio Inc.), pUB110 (Mckenzie, T. et al., 1986, Plasmid 15(2): 93-103), pBR322 (Takara Bio Inc.), pRS403 (Stratagene), pMW218/219 (Nippon Gene Co., Ltd.), pRI-based vectors such as pRI909/910 (Takara Bio Inc.), pBI-based vectors (Clontech), IN3-based vectors (Inplanta Innovations Inc.), pPTR1/2 (Takara Bio Inc.), pDJB2 (D. J. Ballance et al., Gene, 36, 321-331, 1985), pAB4-1 (van Hartingsveldt W et al., Mol Gen Genet, 206, 71-75, 1987), pLeu4 (M. I. G. Roncero et al., Gene, 84, 335-343, 1989), pPyr225 (C. D. Skory et al., Mol Genet Genomics, 268, 397-406, 2002), pFG1 (Gruber, F. et al., Curr Genet, 18, 447-451, 1990), and the like.

Examples of the DNA fragment containing the polynucleotide encoding each enzyme gene include PCR-amplified DNA fragments and restriction enzyme-cleaved DNA fragments. Preferably, the DNA fragment can be an expression cassette containing the polynucleotide of the present invention and a control region operably linked thereto.

The control region contained in the above vector or DNA fragment is a sequence for expressing the each enzyme gene of the present invention in a host into which the vector or DNA fragment has been introduced. Examples include expression control regions such as promoters and terminators, replication origins, and the like. The type of control region can be appropriately selected depending on the type of host cell into which the vector or DNA fragment is to be introduced. If necessary, the vector or DNA fragment may further have a selection marker, such as antibiotic-resistant gene or amino acid synthesis-related gene.

The control region contained in the above vector or DNA fragment is preferably a control region operably linked upstream of the polygalacturonase gene (b1), pectin lyase gene (b2), and one or more genes selected from the group consisting of arabinofuranosidase gene and galactanase gene (c), and having the ability to constitutively express or highly express downstream genes (so-called strong control region). Examples of the strong control region include cbh1 promoter, cbh2 promoter, egl1 promoter, xyn1 promoter, xyn2 promoter, xyn3 promoter, and the like of *Trichoderma reesei.*

Further examples of the strong control region include, but are not limited to, the control region of the rRNA operon, the control region of a gene encoding ribosome protein, and the like.

The target polynucleotide and control region contained in the above vector or DNA fragment may be introduced into the nucleus of the host, but may be introduced into the host genome. Alternatively, the target polynucleotide contained in the above vector or DNA fragment may be directly introduced into the host genome and operably linked to a highly expressed promoter on the genome. The means for introducing the polynucleotide into the genome may be, for example, a homologous recombination method.

To introduce the vector or DNA fragment into the host cell, a general method of transformation, such as an electroporation method, a transformation method, a transfection method, a conjugation method, a protoplast method, a particle gun method, or an Agrobacterium method, can be used.

The recombinant filamentous fungus into which the target vector or DNA fragment has been introduced can be selected using a selection marker. For example, when the selection marker is an antibiotic-resistant gene, the transformed cell into which the target vector or DNA fragment has been introduced can be selected by culturing cells in a medium supplemented with the antibiotic. Further, for example, when the selection marker is an amino acid synthesis-related gene, after the gene is introduced into an amino acid auxotrophic filamentous fungus strain, the filamentous fungus strain into which the target vector or DNA fragment has been introduced can be selected using the presence or absence of amino acid auxotrophy as an indicator. Alternatively, the introduction of the target vector or DNA fragment can also be confirmed by examining the DNA sequence of the recombinant strain by PCR or the like.

With the above procedure, a recombinant filamentous fungus into which the polygalacturonase gene (b1), pectin lyase gene (b2), and one or more genes selected from the group consisting of arabinofuranosidase gene and galactanase gene (c) of the present invention have been introduced can be produced. The recombinant filamentous fungus of the present invention has the ability to produce cellulase, homogalacturonan-degrading enzyme and arabinofuranosidase and/or galactanase.

The filamentous fungus used as the host (parent strain) in the present invention is not limited as long as it is a fungus which produces cellulase as a biomass saccharifying enzyme, and is a filamentous fungus of a different genus or species from the polygalacturonase gene (b1), pectin lyase gene (b2), and one or more genes selected from the group consisting of arabinofuranosidase gene and galactanase gene (c) to be introduced. Examples include filamentous fungi belonging to the phyla *Eumycota* and *Oomycota.* Specific examples of such filamentous fungi include those of the genera *Trichoderma, Aspergillus, Penicillium, Neurospora, Fusarium, Chrysosporium, Humicola, Emericella, Hypocrea, Acremonium, Chrysosporium, Myceliophthora, Piromyces, Talaromyces, Thermoascus, Thielavia,* and the like. Preferred among these are filamentous fungi of the genus *Trichoderma.*

Examples of filamentous fungi of the genus *Trichoderma* include *Trichoderma reesei, Trichoderma longibrachiatum, Trichoderma harzianum, Trichoderma koningii, Trichoderma viride,* and the like; preferably *Trichoderma reesei,* and more preferably *Trichoderma reesei* PCD-10 strain (FERM P-8172) and *Trichoderma reesei* PC-3-7 strain (ATCC66589).

The filamentous fungus as the parent strain may be a wild-type strain, a strain artificially bred from the wild-type strain, or a variant strain (variant) or mutant in which the nucleotide sequence in its genome has been substituted, added, deleted, or modified.

Preferred examples of the recombinant filamentous fungus of the present invention include recombinant filamentous fungi obtained by introducing PgaB gene of *Aspergillus niger,* PelD gene of *Aspergillus niger,* and Abf gene of *Aspergillus niger* or Gal gene of *Aspergillus niger* into *Trichoderma reesei* PC-3-7 strain (ATCC66589) and its variants. Specific examples include the strains disclosed in the Examples described later.

The above recombinant filamentous fungus is cultured in the presence of a cellulase inducer, an enzyme is produced and accumulated in the culture, and the enzyme is collected from the culture, whereby an enzyme composition containing (A) cellulase, (B) a homogalacturonan-degrading enzyme containing polygalacturonase and pectin lyase, and (C) one or more members selected from the group consisting of arabinofuranosidase and galactanase can be produced.

The "cellulase inducer" as mentioned herein is not limited as long as it is a substance which induces the production of cellulase by cellulase-producing filamentous fungi. Examples include cellulose; sophorose; and compounds selected from the group consisting of cello-oligosaccharides, such as cellobiose, cellotriose, cellotetraose, cellopentaose, and cellohexaose.

Cellulose includes polymers in which glucose is polymerized through β-1,4-glucosidic bonds and derivatives thereof. The degree of polymerization of glucose is not particularly limited. Examples of the derivatives include carboxymethylated, aldehydated, or esterified derivatives. Further, cellulose may be β-glucoside, which is a glycoside, lignocellulose, which is a complex with lignin and/or hemicellulose, or a complex with pectin or the like. Cellulose may be crystalline cellulose or non-crystalline cellulose.

The cellulase inducer can be added by any method, such as bulk addition (batch method), divided addition (fed-batch method), or continuous addition (feed method). The amount of cellulase inducer added to the medium may be an amount which allows the filamentous fungus of the present invention to induce the production of cellulase and pectinase, and the amount differs depending on the addition method; however, the total amount is preferably 0.1 mass% or more, more preferably 0.5 mass% or more, and more preferably 1 mass% or more, and is preferably 40 mass% or less, more preferably 35 mass% or less, and more preferably 30 mass% or less, based on the medium. The total amount is also preferably from 0.1 to 40 mass%, more preferably from 0.5 to 35 mass%, and more preferably from 1 to 30 mass%.

Of these, the amount of cellulase inducer added by bulk addition is preferably 0.1 mass% or more, more preferably 0.5 mass% or more, and more preferably 1 mass% or more, and is preferably 16 mass% or less, more preferably 14 mass% or less, and more preferably 12 mass% or less. The amount of cellulase inducer is also preferably from 0.1 to 16 mass%, more preferably from 0.5 to 14 mass%, and more preferably from 1 to 12 mass%.

The medium used in the method of the present invention may be a synthetic medium or a natural medium as long as it contains nutrients necessary for the growth of the filamentous fungus of the present invention and the production of cellulase and pectinase, such as carbon sources, nitrogen sources, inorganic salts, and vitamins.

The carbon source may be any carbon source which can be utilized by the recombinant filamentous fungus of the present invention. Specific examples include the cellulase inducers mentioned above, as well as carbohydrates such as glucose and fructose, alcohols such as ethanol and glycerol, organic acids such as acetic acid, and the like. These can be used singly or in combination of two or more. The carbon source is preferably a carbohydrate such as glucose or fructose, and more preferably glucose. The amount of glucose added in that case is preferably 0.1 mass% or more, more preferably 0.5 mass% or more, and more preferably 2.5 mass% or more, and is preferably 15 mass% or less, more preferably 10 mass% or less, and more preferably 5 mass% or less, based on the medium. The amount of glucose is also preferably from 0.1 to 15 mass%, more preferably from 0.5 to 10 mass%, and more preferably from 2.5 to 5 mass%. As for the amounts of cellulase inducer and glucose in the medium, the mass ratio is preferably from 10:1 to 1:1, and more preferably from 4:1 to 2:1.

Examples of the nitrogen source include ammonia, ammonium salts such as ammonium sulfate, nitrogen compounds such as amines, natural nitrogen sources such as peptone and soy hydrolysate, and the like.

Examples of inorganic salts include potassium phosphate, magnesium sulfate, sodium chloride, ferrous sulfate, potassium carbonate, and the like.

Examples of vitamins include biotin, thiamine, and the like. Further, if necessary, substances required for the growth of the recombinant filamentous fungus of the present invention can be added.

Culture is preferably carried out under aerobic conditions, such as shaking culture or aerated agitation culture. The culture temperature is preferably 10°C or more, more preferably 20°C or more, and more preferably 25°C or more, and is preferably 50°C or less, more preferably 42°C or less, and more preferably 35°C or less. The culture temperature is also preferably from 10 to 50°C, more preferably from 20 to 42°C, and more preferably from 25 to 35°C.

The pH during culturing is from 3 to 9, and preferably from 4 to 5. The culture time is from 10 hours to 10 days, and preferably from 2 to 7 days.

After the completion of culturing, the culture is collected, and optionally subjected to fungal cell crushing treatment using ultrasound, pressurization, or the like, followed by solid-liquid separation by filtration, centrifugation, or the like, and then ultrafiltration, salting-out, dialysis, chromatography, and the like are appropriately combined, whereby an enzyme composition containing (A) cellulase, (B) a homogalacturonan-degrading enzyme containing polygalacturonase and pectin lyase, and (C) one or more members selected from the group consisting of arabinofuranosidase and galactanase can be obtained. The degree of separation and purification is not particularly limited. The culture supernatant and its crudely separated and purified product itself can also be used as the enzyme composition of the present invention.

### (3. Saccharification of Plant Biomass)

Due to the use of the enzyme composition of the present invention, plant biomass can be degraded and saccharified to produce monosaccharides. Specifically, the enzyme composition of the present invention and biomass are allowed to contact (coexist) in an aqueous medium and heated while stirring or shaking to saccharify the plant biomass, thereby producing monosaccharides. In this case, the viscosity of slurry after saccharification can be reduced by 50% or more, preferably 60% or more, and more preferably 70% or more, compared to the case of saccharification using cellulase alone. That is, the enzyme composition of the present invention makes it possible to prevent an increase in viscosity and increase the saccharification efficiency in the saccharification of plant biomass such as cassava containing a large amount of pectin. Therefore, the enzyme composition of the present invention can be used as an agent for reducing the viscosity of plant biomass, and can be used for reducing the viscosity of plant biomass. That is, the enzyme composition of the present invention can be brought into contact with plant biomass to reduce the viscosity of the plant biomass.

The viscosity is measured, for example, by a B-type viscometer at a solid concentration of 10 to 20 mass% at 50°C.

In the saccharification of plant biomass, the pH and temperature of the reaction solution each may be within the range in which cellulase and pectinase are not inactivated. In general, when the reaction is performed at normal pressure, the temperature is within the range from 5 to 95°C, and the pH is within the range from 1 to 11.

The plant biomass saccharification process may be carried out batchwise or continuously.

### (4. Production of Ethanol)

Ethanol can be produced by adding an ethanol fermentation microorganism to sugar-containing liquid obtained by saccharifying plant biomass, followed by fermentation. Examples of known ethanol fermentation microorganisms include yeast of the genus *Saccharomyces,* yeast of the genus *Kluyveromyces,* bacteria of the genus *Zymomonas,* and the like, but are not limited thereto. Fermentation is performed, for example, for 24 to 96 hours at a temperature of 26 to 34°C at a pH of 3 to 6, and preferably at a pH of around 4 to 5. The saccharification step and the fermentation step may be performed separately, or may be performed at the same time, i.e., simultaneous saccharification and fermentation (SSF). SSF is performed by adding yeast and enzymes together, and performed, for example, for 24 to 96 hours at a temperature of 26 to 34°C at a pH of 3 to 6, and preferably at a pH of around 4 to 5. When performing SSF, a pre-saccharification step may be performed at a temperature exceeding 50°C just before fermentation. Further details about the methods for performing saccharification and fermentation are well known to a person skilled in the art.

The present invention also includes the following as exemplary embodiments. However, the present invention is not limited to these embodiments.
<1> An enzyme composition comprising the following (A), (B), and (C):
   (A) cellulase;
   (B) a homogalacturonan-degrading enzyme containing polygalacturonase and pectin lyase; and
   (C) one or more members selected from the group consisting of arabinofuranosidase and galactanase.
<2> The enzyme composition of <1>, for saccharification of plant biomass.
<3> The enzyme composition of <2>, wherein a viscosity of the saccharified plant biomass is reduced compared to a case of saccharification using cellulase alone.
<4> The enzyme composition of <3>, wherein a slurry viscosity of the plant biomass is reduced by 50% or more, preferably 60% or more, and more preferably 70% or more, compared to a case of saccharification using cellulase alone.
<5> The enzyme composition of any one of <1> to <4>, which has a cellulase activity of 2.0 U or more, preferably 2.2 U or more, and more preferably 2.4 U or more, and of 5.0 U or less, preferably 4.0 U or less, and more preferably 3.6 U or less, or from 2.0 to 5.0 U, preferably from 2.2 to 4.0 U, and more preferably from 2.4 to 3.6 U, per mg of protein in the enzyme composition.
<6> The enzyme composition of any one of <1> to <5>, which has a polygalacturonase activity of 30 U or more, preferably 60 U or more, more preferably 90 U or more, and more preferably 150 U or more, and of 1 200 U or less, preferably 600 U or less, and more preferably 450 U or less, or preferably from 30 to 1 200 U, more preferably from 60 to 600 U, more preferably from 90 to 450 U, and more preferably from 150 to 450 U, per mg of protein in the enzyme composition.
<7> The enzyme composition of any one of <1> to <6>, which has a pectin lyase activity of 1 U or more, preferably 1.5 U or more, and more preferably 1.7 U or more, and of 27 U or less, preferably 16 U or less, and more preferably 10 U or less, or preferably from 1 to 27 U, more preferably from 1.5 to 16 U, and more preferably from 1.7 to 10 U, per mg of protein in the enzyme composition.
<8> The enzyme composition of any one of <1> to <7>, which has an arabinofuranosidase activity of 0.1 U or more, preferably 1 U or more, and more preferably 3 U or more, and of 50 U or less, preferably 25 U or less, and more preferably 15 U or less, or preferably from 0.1 to 50 U, more preferably from 1 to 25 U, and more preferably from 3 to 15 U, per mg of protein in the enzyme composition.
<9> The enzyme composition of any one of <1> to <8>, which has a galactanase activity of 3 U or more, preferably 5 U or more, and more preferably 20 U or more, and of 250 U or less, preferably 100 U or less, and more preferably 80 U or less, or preferably from 3 to 250 U, more preferably from 5 to 100 U, and more preferably from 20 to 80 U, per mg of protein in the enzyme composition.
<10> The enzyme composition of any one of <1> to <9>, wherein a content ratio of the homogalacturonan-degrading enzyme (B) and one or more members selected from the group consisting of arabinofuranosidase and galactanase (C) ((B)+(C)) per protein in the enzyme composition is preferably 1 or more, more preferably 5 or more, and more preferably 10 or more, and is preferably 50 or less, more preferably 45 or less, and more preferably 40 or less, or is preferably from 1 to 50, more preferably from 5 to 45, and more preferably from 10 to 40.
<11> A method for saccharifying plant biomass, comprising bringing the enzyme composition of any one of <1> to <10> into contact with plant biomass.
<12> A method for producing sugar from plant biomass, comprising bringing the enzyme composition of any one of <1> to <10> into contact with plant biomass.
<13> A method for producing ethanol from plant biomass, comprising bringing the enzyme composition of any one of <1> to <10> into contact with plant biomass.
<14> The method of any one of <11> to <13>, wherein the plant biomass is pectin-containing plant biomass.
<15> The method of any one of <11> to <13>, wherein the plant biomass is cassava or a residue thereof.
<16> The method of <14> or <15>, wherein a viscosity of the plant biomass which has been brought into contact with the enzyme composition of any one of <1> to <10> is reduced compared to a case of contact using cellulase alone.
<17> A recombinant filamentous fungus comprising (b1) a polygalacturonase gene, (b2) a pectin lyase gene, and (c) one or more genes selected from the group consisting of arabinofuranosidase gene and galactanase gene which are introduced thereto.
<18> A method for producing the enzyme composition of any one of <1> to <10>, comprising culturing the recombinant filamentous fungus of <17>.
<19> The recombinant filamentous fungus of <17>, wherein the polygalacturonase gene (b1) is PgaB gene, the pectin lyase gene (b2) is PelD gene, and (c) is Abf gene or Gal gene.
<20> The method of <18>, wherein the polygalacturonase gene (b1) is PgaB gene, the pectin lyase gene (b2) is PelD gene, and (c) is Abf gene or Gal gene.
<21> An agent for reducing a viscosity of plant biomass comprising the enzyme composition of any one of <1>, <2>, and <5> to <10> as an active ingredient.
<22> A method for reducing a viscosity of plant biomass, comprising bringing the enzyme composition of any one of <1>, <2>, and <5> to <10> into contact with plant biomass.
<23> Use of the enzyme composition of any one of <1>, <2>, and <5> to <10> for reducing plant biomass viscosity.

### Examples

### <Example 1: Preparation of cellulase>

Cellulase was obtained by culturing *Trichoderma reesei* E1AB1 strain (Enzyme and Microbial Technology, Volume 82, January 2016, pages 89-95).

Culture was carried out as follows. The spores of the strain were inoculated to 2×10⁵ cells/mL into an enzyme production medium (3% KC Flock W-400G (Nippon Paper Industries Co., Ltd.), 0.14% (NH₄)₂SO₄, 1.28% diammonium hydrogen citrate, 0.2% KH₂PO₄, 0.03% Cacl₂·2H₂O, 0.03% MgSO₄·7H₂O, 0.1% Bacto Peptone, 0.05% Bacto Yeast Extract, 0.1% Tween 80, 0.1% Trace element, 50 mM tartrate buffer (pH 4.0); all percentages are w/v%) and cultured by shaking at 28°C for 5 days.

The composition of the Trace element is as follows: 6 mg H₃BO₃, 26 mg (NH₄)6Mo₇O₂₄·4H₂O, 100 mg FeCl₃·6H₂O, 40 mg CuSO₄·5H₂O, 8 mg MnCl₂·4H₂O, and 200 mg ZnCl₂ were made up to 100 mL with distilled water. After the obtained culture was centrifuged, the supernatant was filtered, and the resultant was used as cellulase for the following examination.

### <Example 2: Preparation of pectinase>

### (1) Synthesis of cDNA of Aspergillus niger NBRC 105649 strain

*A. niger* NBRC 105649 strain was purchased from the National Institute of Technology and Evaluation, Biological Resource Center. The NBRC 105649 strain was inoculated into a pectin medium (1% Pectin, from Citrus (Wako Pure Chemical), 0.14% (NH₄)₂SO₄, 0.2% KH₂PO₄, 0.03% Cacl₂·2H₂O, 0.03% MgSO₄·7H₂O, 0.1% Bacto Peptone, 0.05% Bacto Yeast Extract, 0.1% Trace element, 50 mM tartrate buffer (pH 4.0). Shaking culture was performed at 28°C for 3 days, and the cultured fungal cells were collected using a Miracloth (Millipore), then frozen in liquid nitrogen, and crushed using a Multi-Beads Shocker (Yasui Kikai Corporation). In this case, a metal cone was used as the crushing medium, and crushing was performed at 1 700 rpm for 30 seconds. RNA was extracted from the crushed fungal cells using the RNeasy Mini Kit (Qiagen) in accordance with the protocol. Thereafter, the RNA solution was subjected to the SuperScript III First-Strand Synthesis System for RT-PCR (Thermo Fisher Scientific), thereby synthesizing the cDNA of the NBRC 105649 strain.

### (2) Production of expression vectors for Pga, Pel, Abf, Gal, and Bga1

Using *Pichia* expression vector pD912 (ATUM) as a template, PCR was performed using the Fw primer 1 (SEQ ID NO: 15) and Rv primer 1 (SEQ ID NO: 16) shown in Table 1 to amplify a fragment (A). Using the cDNA of the NBRC 105649 strain as a template, PCR was performed using the Fw primer 2 (SEQ ID NO: 17) and Rv primer 2 (SEQ ID NO: 18) shown in Table 1 to amplify an amplified fragment (B). The obtained DNA fragments (A) and (B) were treated in accordance with the protocol of the In-Fusion HD Cloning Kit (Takara Bio Inc.), thereby constructing ligated plasmid pD912-Pga.

Using the cDNA of the NBRC 105649 strain as a template, PCR was performed using the Fw primer 3 (SEQ ID NO: 19) and Rv primer 3 (SEQ ID NO: 20) shown in Table 1 to amplify an amplified fragment (C). The obtained DNA fragments (A) and (C) were treated in accordance with the protocol of the In-Fusion HD Cloning Kit, thereby constructing ligated plasmid pD912-Pel.

Using the cDNA of the NBRC 105649 strain as a template, PCR was performed using the Fw primer 4 (SEQ ID NO: 21) and Rv primer 5 (SEQ ID NO: 22) shown in Table 1 to amplify an amplified fragment (D). The obtained DNA fragments (A) and (D) were treated in accordance with the protocol of the In-Fusion HD Cloning Kit, thereby constructing ligated plasmid pD912-AnAbf.

Using the cDNA of the NBRC 105649 strain as a template, PCR was performed using the Fw primer 5 (SEQ ID NO: 23) and Rv primer 5 (SEQ ID NO: 24) shown in Table 1 to amplify an amplified fragment (E). The obtained DNA fragments (A) and (E) were treated in accordance with the protocol of the In-Fusion HD Cloning Kit, thereby constructing ligated plasmid pD912-AnGal.

Using the cDNA of the NBRC 105649 strain as a template, PCR was performed using the Fw primer X (SEQ ID NO: 48) and Rv primer X (SEQ ID NO: 49) shown in Table 1 to amplify an amplified fragment (I). The obtained DNA fragments (A) and (I) were treated in accordance with the protocol of the In-Fusion HD Cloning Kit, thereby constructing ligated plasmid pD912-Bga1.

**[Table 1]**

| Primers for amplifying gene fragments | | |
|---|---|---|
| Primer name | Sequence | SEQ ID NO: |
| Fw primer 1 | TAAAGGGGCGGCCGCTCAAGAG | 15 |
| Rv primer 1 | AGCTTCGGCCTCTCTTTTCT | 16 |
| Fw primer 2 | AGAGAGGCCGAAGCTGCTCCTCTCGAGAAGCGCTC | 17 |
| Rv primer 2 | GCGGCCGCCCCTTTATTAATCGCTGCAGGAAGCGC | 18 |
| Fw primer 3 | AGAGAGGCCGAAGCTGTCGGTGTCTCCGGCACTCC | 19 |
| Rv primer 3 | GCGGCCGCCCCTTTATTACAGGTTACCCTGACCAG | 20 |
| Fw primer 4 | AGAGAGGCCGAAGCTATCTCCTTGAAGGTCTCCAC | 21 |
| Rv primer 4 | GCGGCCGCCCCTTTACTAGTTCGCCGCCAGGACAG | 22 |
| Fw primer 5 | AGAGAGGCCGAAGCTGCCCTGACCTACCGCGGGGC | 23 |
| Rv primer 5 | GCGGCCGCCCCTTTATCAGAGGTCCTCAAACACGG | 24 |
| Fw primer 17 | AGAGAGGCCGAAGCTGCCCAGAACCAGACGGAGAC | 48 |
| Rv primer 17 | GCGGCCGCCCCTTTATCAAGCAAACTTCAACCTCT | 49 |

### (3) Production of transformant

Each of the target gene expression vectors produced in (2) was transformed into *Pichia pastoris* PPS-90102 (ATUM) in accordance with the ATUM's protocol.

### (4) Culture of transformant

*P. pastoris* was cultured in the following manner. Each transformant was inoculated into a BMD 1% medium (0.2 M potassium phosphate (pH 6.0), 13.4 g/L yeast nitrogen base, 0.4 mg/L biotin, 1.1% glucose) and cultured by shaking at 28°C. After the fungal cells were grown to a certain amount, methanol was added to induce expression, and cultures obtained after 3 to 5 days were each confirmed for the enzyme expression level by SDS-PAGE. Then, the cultures were each centrifuged and then filtered to obtain culture supernatants. The obtained culture supernatants were concentrated and used as enzyme solutions for various pectinases (Pga, Pel, Abf, and Gal).

### <Example 3> Quantification of protein

The protein concentration of the enzyme solution was quantified by the Bradford method. In the Bradford method, using the Quick Start Protein Assay (BioRad), the protein concentration was calculated based on a calibration curve using bovine serum albumin as a standard protein.

### <Example 4> Enzyme unit measurement

### (1) Pectate lyase activity

The pectate lyase activity was measured in such a manner that 20 µL of an enzyme solution appropriately diluted with milliQ water was added to 80 µL of a substrate solution prepared by mixing 25 µL of 1% (w/v) polygalacturonic acid (polygalacturonic acid sodium salt, Sigma-Aldrich), 10 µL of 0.5 M sodium acetate buffer (pH 4.0), and 45 µL of milliQ water, and the resulting mixture was reacted at 50°C for 5 minutes, after which 100 µL of 50 mM hydrochloric acid aqueous solution was added to stop the enzymatic reaction. After stirring and mixing, the absorbance at 235 nm was measured. The blank was prepared by adding a hydrochloric acid aqueous solution to a reaction solution treated without adding an enzyme solution, and then adding an enzyme solution. The amount of unsaturated oligogalacturonide produced was determined using the molar molecular absorption coefficient of unsaturated digalacturonide, i.e., 4 600 M⁻¹cm⁻¹. One unit of enzyme (1 U) was defined as the amount of enzyme which produces unsaturated oligogalacturonide equivalent to 1 µmol of unsaturated digalacturonide per minute under the above reaction conditions.

### (2) Polygalacturonase activity

The polygalacturonase activity was measured in such a manner that 20 µL of an enzyme solution appropriately diluted with milliQ water was added to 80 µL of a substrate solution prepared by mixing 25 µL of 1% (w/v) polygalacturonic acid (polygalacturonic acid sodium salt, Sigma-Aldrich), 10 µL of 0.5 M sodium acetate buffer (pH 4.0), and 45 µL of milliQ water, and the resulting mixture was reacted at 50°C for 5 minutes, after which 100 µL of 3,5-dinitrosalicylic acid reagent was added, and the color development of reducing sugar was performed at 99°C for 10 minutes. After quenching in ice water, the absorbance at 540 nm was measured, and the amount of reducing sugar produced was determined. The blank was prepared by adding a 3,5-dinitrosalicylic acid reagent to a reaction solution treated without adding an enzyme, then adding an enzyme solution, and performing color development in the same manner. One unit of enzyme (1 U) was defined as the amount of enzyme which releases reducing sugar equivalent to 1 µmol of D-galacturonic acid per minute under the above reaction conditions. However, when polygalacturonase and pectin lyase were both present in the enzyme solution, the polygalacturonase activity was determined by subtracting the unit of pectin lyase obtained in (1) from the unit calculated from the amount of reducing sugar.

### (3) Arabinofuranosidase activity

The arabinofuranosidase activity was measured in such a manner that 20 µL of an enzyme solution appropriately diluted with milliQ water was added to 80 µL of a substrate solution prepared by mixing 20 µL of 5 mM 4-nitrophenyl-α-L-arabinofuranoside (Sigma-Aldrich), 10 µL of 0.5 M sodium acetate buffer (pH 4.0), and 40 µL of milliQ water, and the resulting mixture was reacted at 50°C for 5 minutes, after which 100 µL of 0.4 M sodium carbonate was added, and the absorbance at 420 nm was measured. The blank was prepared by adding a sodium carbonate solution to a reaction solution treated without adding an enzyme solution, and then adding an enzyme solution inactivated at 99°C. One unit of enzyme (1 U) was defined as the amount of enzyme which produces 1 µmol of 4-nitrophenol per minute under the above reaction conditions.

### (4) Galactanase activity

The galactanase activity was measured in such a manner that 20 µL of an enzyme solution appropriately diluted with milliQ water was added to 80 µL of a substrate solution prepared by mixing 25 µL of 1% (w/v) galactan (potato, Megazyme), 10 µL of 0.5 M sodium acetate buffer (pH 4.0), and 45 µL of milliQ water, and the resulting mixture was reacted at 50°C for 5 minutes, after which 100 µL of 3,5-dinitrosalicylic acid reagent was added, and the color development of reducing sugar was performed at 99°C for 10 minutes. After quenching in ice water, the absorbance at 540 nm was measured, and the amount of reducing sugar produced was determined. The blank was prepared by adding a 3,5-dinitrosalicylic acid reagent to a reaction solution treated without adding an enzyme, then adding an enzyme solution, and performing color development in the same manner. One unit of enzyme (1 U) was defined as the amount of enzyme which releases reducing sugar equivalent to 1 µmol of galactose per minute under the above reaction conditions.

### (5) Cellulase activity

The cellulase activity was measured by the microplate scale filter paper degradation activity test method reported in Microplate-based filter paper assay to measure total cellulase activity (Biotechnology and Bioengineering. 2004 DEC 30; 88(7): 832-7. DOI: 10.1002/BIT.20286). 20 µL of appropriately diluted sample was added to 40 µL of citrate buffer (50 mM, pH 4.8) containing filter paper (Whatman NO. 1, GE Healthcare) cut to 7 mm in diameter, and the reaction was carried out at 50°C for 1 hour. A sample without filter paper was prepared in the same manner as a blank sample. Then, 120 µL of DNS solution was added to stop the reaction, followed by heating at 95°C for 5 minutes and cooling in ice water for 5 minutes. 36 µL of the obtained solution was mixed with 160 µL of milliQ water, the absorbance at 540 nm of the mixed liquid was measured, and the increase in absorbance was calculated by subtracting the blank. Next, the amount of reducing sugar produced in the reaction solution was calculated in terms of the amount of glucose using a calibration curve generated with gradual dilution of glucose concentration and absorbance at 540 nm. The same procedure was performed with samples of different dilution rates to determine the dilution rate of the sample required to produce reducing sugar equivalent to 0.08 mg glucose/20 µL cellulase solution, and the filter paper degradation activity (FPU/mL) of the sample before dilution was calculated. Further, the cellulase concentration was measured by the method of Example 4, and the display unit of cellulase activity was converted to FPU/mg-PROTEIN. As for the activity unit, the enzyme activity which produces reducing sugar equivalent to 1 µmol of glucose per minute was defined as "1 U."

### <Example 5> Measurement of viscosity

The viscosity was measured using a tuning-fork vibration viscometer (SV-10H, produced by A&D Co., Ltd.).

### Test Examples: Saccharification of cassava residue

### <Test Example 1 - Comparative Example>

Cassava residue was weighed in a 100-mL baffled Erlenmeyer flask so that the dry weight was 4 g, and 0.002 mg-protein/g-substrate of α-amylase and Milli-Q water were added so that the substrate concentration was 15 wt%. Thereafter, the content of the baffled flask was thoroughly mixed, and the flask was sealed with a cotton plug and covered with aluminum foil, followed by gelatinization treatment at 90°C for 2 hours (Autoclave LSX-700, produced by Tomy Seiko Co., Ltd.). Thereafter, the flask was cooled to 50°C using a shaker (PRXY-30-R-3F, produced by PRECI) set at 50°C. Then, glucoamylase was added so that the total amount was 0.05 mg-protein/g-substrate, followed by reaction at 50°C at 200 rpm for 18 hours. A liquid state was not obtained after saccharification, and the viscosity could not be measured.

### <Test Example 2 - Comparative Example>

The same examination as in Test Example 1 was performed, except that 0.2 mg-protein/g-substrate of the enzyme composition 1 shown in Table 2 was further added to the substrate after gelatinization treatment. The slurry after saccharification was not in a liquid state, and the viscosity could not be measured.

### <Test Example 3 - Comparative Example>

The same examination as in Test Example 1 was performed, except that 0.2 mg-protein/g-substrate of the enzyme composition 2 shown in Table 2 was further added to the substrate after gelatinization treatment. The viscosity value of the slurry after saccharification was 84 mPa·s.

### <Test Example 4 - Comparative Example>

The same examination as in Test Example 1 was performed, except that 0.2 mg-protein/g-substrate of the enzyme composition 3 shown in Table 2 was further added to the substrate after gelatinization treatment. The viscosity value of the slurry after saccharification was 79 mPa·s.

### <Test Example 5 - Comparative Example>

The same examination as in Test Example 1 was performed, except that 0.2 mg-protein/g-substrate of the enzyme composition 4 shown in Table 2 was further added to the substrate after gelatinization treatment. The viscosity value of the slurry after saccharification was 459 mPa·s.

### <Test Example 6>

The same examination as in Test Example 1 was performed, except that 0.16 mg-protein/g-substrate of the enzyme composition 5 shown in Table 3 was further added to the substrate after gelatinization treatment. The viscosity value of the slurry after saccharification was 26.8 mPa·s.

### <Test Example 7>

The same examination as in Test Example 1 was performed, except that 0.16 mg-protein/g-substrate of the enzyme composition 6 shown in Table 3 was further added to the substrate after gelatinization treatment. The viscosity value of the slurry after saccharification was 16.6 mPa·s.

### <Test Example 8>

The same examination as in Test Example 1 was performed, except that 0.16 mg-protein/g-substrate of the enzyme composition 7 shown in Table 3 was further added to the substrate after gelatinization treatment. The viscosity value of the slurry after saccharification was 14.5 mPa·s.

### <Test Example 9>

The same examination as in Test Example 1 was performed, except that 0.16 mg-protein/g-substrate of the enzyme composition 8 shown in Table 3 was further added to the substrate after gelatinization treatment. The viscosity value of the slurry after saccharification was 17.3 mPa·s.

### <Test Example 10>

The same examination as in Test Example 1 was performed, except that 0.15 mg-protein/g-substrate of the enzyme composition 9 shown in Table 4 was further added to the substrate after gelatinization treatment. The viscosity value of the slurry after saccharification was 47.3 mPa·s.

### <Test Example 11>

The same examination as in Test Example 1 was performed, except that 0.15 mg-protein/g-substrate of the enzyme composition 10 shown in Table 4 was further added to the substrate after gelatinization treatment. The viscosity value of the slurry after saccharification was 37.9 mPa·s.

### <Test Example 12>

The same examination as in Test Example 1 was performed, except that 0.15 mg-protein/g-substrate of the enzyme composition 11 shown in Table 4 was further added to the substrate after gelatinization treatment. The viscosity value of the slurry after saccharification was 19.7 mPa·s.

### <Test Example 13>

The same examination as in Test Example 1 was performed, except that 0.15 mg-protein/g-substrate of the enzyme composition 12 shown in Table 4 was further added to the substrate after gelatinization treatment. The viscosity value of the slurry after saccharification was 17.3 mPa·s.

### <Test Example 14>

The same examination as in Test Example 1 was performed, except that 0.15 mg-protein/g-substrate of the enzyme composition 13 shown in Table 4 was further added to the substrate after gelatinization treatment. The viscosity value of the slurry after saccharification was 23.1 mPa·s.

### <Test Example 15>

The same examination as in Test Example 1 was performed, except that 0.15 mg-protein/g-substrate of the enzyme composition 14 shown in Table 4 was further added to the substrate after gelatinization treatment. The viscosity value of the slurry after saccharification was 23.8 mPa·s.

### <Test Example 16>

The same examination as in Test Example 1 was performed, except that 0.15 mg-protein/g-substrate of the enzyme composition 15 shown in Table 4 was further added to the substrate after gelatinization treatment. The viscosity value of the slurry after saccharification was 19.9 mPa·s.

### <Test Example 17>

The same examination as in Test Example 1 was performed, except that 0.15 mg-protein/g-substrate of the enzyme composition 16 shown in Table 4 was further added to the substrate after gelatinization treatment. The viscosity value of the slurry after saccharification was 39.0 mPa·s.

### <Test Example 18 - Comparative Example>

The same examination as in Test Example 1 was performed, except that 0.14 mg-protein/g-substrate of the enzyme composition 17 shown in Table 5 was further added to the substrate after gelatinization treatment. The viscosity value of the slurry after saccharification was 115 mPa·s.

### <Test Example 19>

The same examination as in Test Example 1 was performed, except that 0.14 mg-protein/g-substrate of the enzyme composition 18 shown in Table 5 was further added to the substrate after gelatinization treatment. The viscosity value of the slurry after saccharification was 71.8 mPa·s.

### <Test Example 20>

The same examination as in Test Example 1 was performed, except that 0.13 mg-protein/g-substrate of the enzyme composition 19 shown in Table 6 was further added to the substrate after gelatinization treatment. The viscosity value of the slurry after saccharification was 49.1 mPa·s.

### <Test Example 21>

The same examination as in Test Example 1 was performed, except that 0.15 mg-protein/g-substrate of the enzyme composition 20 shown in Table 6 was further added to the substrate after gelatinization treatment. The viscosity value of the slurry after saccharification was 47.5 mPa·s.

### <Test Example 22 - Comparative Example>

The same examination as in Test Example 1 was performed, except that 0.15 mg-protein/g-substrate of the enzyme composition 21 containing pectin side chain-degrading enzyme AnBga1 shown in Table 7 was further added to the substrate after gelatinization treatment. The viscosity value of the slurry after saccharification was 1 440 mPa·s. AnBga1 is β-galactosidase, which is an exo-type galactan-degrading enzyme.

### <Test Example 23 - Comparative Example>

The same examination as in Test Example 1 was performed, except that 0.15 mg-protein/g-substrate of the enzyme composition 22 containing pectin side chain-degrading enzyme E-ARABCJ (Megazyme) shown in Table 7 was further added to the substrate after gelatinization treatment. The viscosity value of the slurry after saccharification was 4 010 mPa·s. E-ARABCJ is an endo-type arabinan-degrading enzyme.

### <Test Example 24 - Comparative Example>

The same examination as in Test Example 1 was performed, except that 0.15 mg-protein/g-substrate of the enzyme composition 23 containing pectin side chain-degrading enzyme E-BGLAN (Megazyme) shown in Table 7 was further added to the substrate after gelatinization treatment. The viscosity value of the slurry after saccharification was 530 mPa·s. E-BGLAN is β-galactosidase, which is an exo-type galactan-degrading enzyme.

**[Table 2]**

| | | | Cellulase activity | Pectinase activity | | | | | |
|---|---|---|---|---|---|---|---|---|---|
| Test Example | Enzyme composition No. | Amount of enzyme composition added | FPU | Pga | Pel | Abf | Gal | Pectinase content per protein weight | Viscosity |
| | | mg-protein/g-substrate | FPU/mg-protein | U/mg-protein | U/mg-protein | U/mg-protein | U/mg-protein | % | mPa·s |
| 2 (comparison) | 1 | 0.2 | 4.1 | 0 | 0 | 0 | 0 | 0% | Not measurable |
| 3 (comparison) | 2 | 0.2 | 3.7 | 231.7 | 0 | 0 | 0 | 10% | 84 |
| 4 (comparison) | 3 | 0.2 | 2.9 | 393.8 | 6.9 | 0 | 0 | 30% | 79 |
| 5 (comparison) | 4 | 0.2 | 2.9 | 0 | 15.9 | 0 | 0 | 30% | 459 |

**[Table 3]**

| | | | Cellulase activity | Pectinase activity | | | | | |
|---|---|---|---|---|---|---|---|---|---|
| Test Example | Enzyme composition No. | Amount of enzyme composition added | FPU | Pga | Pel | Abf | Gal | Pectinase content per protein weight | Viscosity |
| | | mg-protein/g-substrate | FPU/mg-protein | U/mg-protein | U/mg-protein | U/mg-protein | U/mg-protein | % | mPa·s |
| 6 | 5 | 0.16 | 2.6 | 434.3 | 6.6 | 5.9 | 0 | 38% | 26.8 |
| 7 | 6 | 0.16 | 2.6 | 434.3 | 6.6 | 0 | 30.9 | 38% | 16.6 |
| 8 | 7 | 0.16 | 2.6 | 434.3 | 6.6 | 1.2 | 24.7 | 38% | 14.5 |
| 9 | 8 | 0.16 | 2.6 | 289.6 | 5 | 5.9 | 46.3 | 38% | 17.3 |

**[Table 4]**

| | | | Cellulase activity | Pectinase activity | | | | | |
|---|---|---|---|---|---|---|---|---|---|
| Test Example | Enzyme composition No. | Amount of enzyme composition added | FPU | Pga | Pel | Abf | Gal | Pectinase content per protein weight | Viscosity |
| | | mg-protein/g-substrate | FPU/mg-protein | U/mg-protein | U/mg-protein | U/mg-protein | U/mg-protein | % | mPa·s |
| 10 | 9 | 0.15 | 2.7 | 308.9 | 7.1 | 6.3 | 0 | 33% | 47.3 |
| 11 | 10 | 0.15 | 2.7 | 308.9 | 7.1 | 0 | 32.9 | 33% | 37.9 |
| 12 | 11 | 0.15 | 2.7 | 308.9 | 7.1 | 1.3 | 26.3 | 33% | 19.7 |
| 13 | 12 | 0.15 | 3 | 154.4 | 3.5 | 3.1 | 49.4 | 27% | 17.3 |
| 14 | 13 | 0.15 | 2.8 | 308.9 | 7.1 | 0.6 | 26.3 | 33% | 23.1 |
| 15 | 14 | 0.15 | 2.8 | 309 | 7.1 | 0.1 | 26.3 | 33% | 23.8 |
| 16 | 15 | 0.15 | 2.7 | 308.9 | 7.1 | 5 | 6.6 | 33% | 19.9 |
| 17 | 16 | 0.15 | 2.8 | 308.9 | 7.1 | 5 | 3.3 | 33% | 39 |

**[Table 5]**

| | | | Cellulase activity | Pectinase activity | | | | | |
|---|---|---|---|---|---|---|---|---|---|
| Test Example | Enzyme composition No. | Amount of enzyme composition added | FPU | Pga | Pel | Abf | Gal | Pectinase content per protein weight | Viscosity |
| | | mg-protein/g-substrate | FPU/mg-protein | U/mg-protein | U/mg-protein | U/mg-protein | U/mg-protein | % | mPa·s |
| 18 (comparison) | 17 | 0.14 | 2.9 | 330.9 | 7.6 | 0 | 0 | 29% | 115 |
| 19 | 18 | 0.14 | 2.9 | 165.5 | 3.8 | 6.7 | 35.3 | 29% | 71.8 |

**[Table 6]**

| | | | Cellulase activity | Pectinase activity | | | | | |
|---|---|---|---|---|---|---|---|---|---|
| Test Example | Enzyme composition No. | Amount of enzyme composition added | FPU | Pga | Pel | Abf | Gal | Pectinase content per protein weight | Viscosity |
| | | mg-protein/g-substrate | FPU/mg-protein | U/mg-protein | U/mg-protein | U/mg-protein | U/mg-protein | % | mPa·s |
| 20 | 19 | 0.13 | 3.2 | 72.4 | 1.7 | 7.4 | 38.6 | 23% | 49.1 |
| 21 | 20 | 0.15 | 3.0 | 30.9 | 7.1 | 5.0 | 32.9 | 27% | 47.5 |

**[Table 7]**

| | | | Cellulase activity | Pectinase activity | | | | Other pectin side chain-degrading enzymes | | |
|---|---|---|---|---|---|---|---|---|---|---|
| Test Example | Enzyme composition No. | Amount of enzyme composition added | FPU | Pga | Pel | Abf | Gal | Enzyme name | Pectinase content per protein weight | Viscosity |
| | | mg-protein/g-substrate | FPU/mg-protein | U/mg-protein | U/mg-protein | U/mg-protein | U/mg-protein | wt% | % | mPa·s |
| 22 (comparison) | 21 | 0.15 | 2.7 | 308.9 | 7.1 | 0.0 | 0.0 | Bga1 5% | 36 | 1440 |
| 23 (comparison) | 22 | 0.15 | 2.7 | 308.9 | 7.1 | 0.0 | 0.0 | E-ARABCJ 5% | 36 | 4010 |
| 24 (comparison) | 23 | 0.15 | 2.7 | 308.9 | 7.1 | 0.0 | 0.0 | E-BGLAN 5% | 36 | 530 |

### <Example 6> Construction of cellulase- and pectinase-producing strains

### (1) Construction of expression vectors for Gal

Using, as a template, a plasmid pUC-eg3 obtained by inserting a region from upstream to downstream of *Trichoderma reesei*-derived eg3 gene (SEQ ID NO: 47) into the cutting site of the HincII restriction enzyme of pUC118 (Takara Bio Inc.), PCR was performed using the Fw primer 6 (SEQ ID NO: 25) and Rv primer 6 (SEQ ID NO: 26) shown in Table 7 to amplify a fragment (A). In addition, using the genomic DNA of *Trichoderma reesei* as a template, PCR was performed using the Fw primer 7 (SEQ ID NO: 27) and Rv primer 7 (SEQ ID NO: 28) shown in Table 7 to amplify an amplified fragment (B). The obtained DNA fragments (A) and (B) were treated in accordance with the protocol of the In-Fusion HD Cloning Kit (Takara Bio Inc.) to construct plasmid pUC-eg3-pyr4 in which pyr4 gene was linked downstream of eg3 gene.

Further, using this pUC-eg3-pyr4 as a template, PCR was performed using the Fw primer 8 (SEQ ID NO: 29) and Rv primer 8 (SEQ ID NO: 30) shown in Table 7 to amplify a fragment (C), and using the genomic DNA of *Trichoderma reesei* as a template, PCR was performed using the Fw primer 9 (SEQ ID NO: 31) and Rv primer 9 (SEQ ID NO: 32) shown in Table 7 to amplify a fragment (D). These fragments were similarly treated by using the In-Fusion HD Cloning Kit to obtain plasmid pUC-Δeg3::Pcbh1-cbh1-pyr4.

Subsequently, using this pUC-Δeg3::Pcbh1-cbh1-pyr4 as a template, PCR was performed using the Fw primer 10 (SEQ ID NO: 33) and Rv primer 10 (SEQ ID NO: 34) shown in Table 7 to amplify a fragment (E), and using the plasmid pUC-eg3 as a template, PCR was performed using the Fw primer 11 (SEQ ID NO: 35) and Rv primer 11 (SEQ ID NO: 36) shown in Table 7 to amplify a fragment (F). These fragments were similarly treated by using the In-Fusion HD Cloning Kit to obtain plasmid pUC-Δeg3::Pcbh1-cbh1-pyr4_1000.

Next, using the pUC-Δeg3::Pcbh1-cbh1-pyr4_1000 as a template, PCR was performed using the Fw primer 7 (SEQ ID NO: 27) and Rv primer 9 (SEQ ID NO: 32) shown in Table 7 to amplify a fragment (G), and using the genomic DNA of *Trichoderma reesei* as a template, PCR was performed using the Fw primer 12 (SEQ ID NO: 37) and Rv primer 12 (SEQ ID NO: 38) shown in Table 8 to amplify a fragment (H). These fragments were similarly treated by using the In-Fusion HD Cloning Kit to obtain plasmid pUC-Δeg3::Pcbh1-cbh1-pyr4 (re) .

Using the pUC-Δeg3::Pcbh1-cbh1-pyr4(re) as a template, PCR was performed using the Fw primer 13 (SEQ ID NO: 39) and Rv primer 13 (SEQ ID NO: 40) shown in Table 7 to amplify a fragment (I), and using the genomic DNA of *Trichoderma reesei* as a template, PCR was performed using the Fw primer 14 (SEQ ID NO: 41) and Rv primer 14 (SEQ ID NO: 42) shown in Table 7 to amplify a fragment (J). These fragments were similarly treated by using the In-Fusion HD Cloning Kit to obtain plasmid pUC-Δeg3::Pxyn1-cbh1-pyr4(re).

Then, using the pUC-Δeg3::Pxyn1-cbh1-pyr4(re) as a template, PCR was performed using the Fw primer 15 (SEQ ID NO: 43) and Rv primer 15 (SEQ ID NO: 44) shown in Table 7 to amplify a fragment (K), and using the cDNA of *Aspergillus niger* NBRC 105649 strain as a template, PCR was performed using the Fw primer 16 (SEQ ID NO: 45) and Rv primer 16 (SEQ ID NO: 46) shown in Table 7 to amplify a fragment (L). These fragments were similarly treated by using the In-Fusion HD Cloning Kit to obtain GAL2 expression plasmid pUC-Δeg3::Pxyn1-AnGAL-pyr4(re).

**[Table 8]**

| Primers for amplifying gene fragments | | |
|---|---|---|
| Primer name | Sequence | SEQ ID NO: |
| Fw primer 6 | ATATCTGGTGGATCTATGCATTATGCGCAAATGCT | 25 |
| Rv primer 6 | ACAACGAGAAAAGAAAACACCTGGAAAGCTTGCCG | 26 |
| Fw primer 7 | TTCTTTTCTCGTTGTCTCACTCT | 27 |
| Rv primer 7 | AGATCCACCAGATATGTCAGGA | 28 |
| Fw primer 8 | TACCACGGCAAAGGTTTCTTTTCTCGTTGTCTCAC | 29 |
| Rv primer 8 | TCGGGCTTCGAACAGGATGGCGTCGCTTTCTAGGC | 30 |
| Fw primer 9 | CTGTTCGAAGCCCGAATGTAG | 31 |
| Rv primer 9 | ACCTTTGCCGTGGTACTGGG | 32 |
| Fw primer 10 | ATGCCCACCATATCTCTGTTCGAAGCCCGAATGTA | 33 |
| Rv primer 10 | TCACGTTTCCAGGTTAGATCCACCAGATATGTCAG | 34 |
| Fw primer 11 | AACCTGGAAACGTGAGATGT | 35 |
| Rv primer 11 | AGATATGGTGGGCATTGCGT | 36 |
| Fw primer 12 | TACCACGGCAAAGGTAACCTGGAAACGTGAGATGT | 37 |
| Rv primer 12 | ACAACGAGAAAAGAATCATGAATATATGCTTGGTA | 38 |
| Fw primer 13 | ATGTATCGGAAGTTGGCCGTC | 39 |
| Rv primer 13 | AGATATGGTGGGCATTGCGT | 40 |
| Fw primer 14 | ATGCCCACCATATCTCAAGTCTTCGTACTCTATCG | 41 |
| Rv primer 14 | AACTTCCGATACATGATGATTATTTGTGCGTGTTT | 42 |
| Fw primer 15 | AGCTCCGTGGCGAAAGCCTG | 43 |
| Rv primer 15 | GATGATTATTTGTGCGTGTT | 44 |
| Fw primer 16 | GCACAAATAATCATCATGATCTACTCTCTGCTTCT | 45 |
| Rv primer 16 | TTTCGCCACGGAGCTTCAGAGGTCCTCAAACACGG | 46 |

### (2) Production of transformant

A strain obtained by expressing pgaB and pelD in a uracil auxotrophic strain of *Trichoderma reesei* E1AB1 strain (Japanese Patent Application No. 2021-154749) was regarded as SP21, and the vectors constructed in (1) above were each transformed into this strain. Introduction was performed by the protoplast PEG method. Transformants were selected in a selective medium with acetamide as the sole nitrogen source (2.0% glucose, 1.1 M sorbitol, 2.0% agar, 0.2% KH₂PO₄, 0.06% CaCl₂.2H₂O, 0.06% MgSO₄.7H₂O, 0.06% acetamide, 0.1% Trace element 2; all percentages are w/v%). The composition of Trace element 2 is as follows: 0.5 g FeSO₄.7H₂O, 0.2 g CoCl₂, 0.16 g MnSO₄·H₂O, and 0.14 g ZnSO₄·7H₂O were made up to 100 mL with distilled water. After the selected transformants were stabilized by succession, strains in which the target genes were stably maintained were further selected by colony PCR. The obtained transformant was regarded as SP21·Gal.

### (3) Enzyme production

The spores of SP21 or SP21·Gal were inoculated to 2×10⁵ cells/mL into an enzyme production medium, and cultured by shaking at 28°C for 5 days. The obtained cultures were each centrifuged, followed by filter filtration to obtain culture supernatants. The obtained culture supernatants were used for the examination as enzyme solutions.

### Test Examples: Saccharification of cassava residues

### <Test Example 21>

The same examination as in Test Example 1 was performed, except that 0.05 mg-protein/g-substrate of glucoamylase and 0.2 mg-protein/g-substrate of the SP21-derived culture supernatant were added to the substrate after gelatinization treatment. The viscosity value of the slurry after saccharification was 60.0 mPa·s.

### <Test Example 22>

The same examination as in Test Example 1 was performed, except that 0.05 mg-protein/g-substrate of glucoamylase and 0.2 mg-protein/g-substrate of the SP21·Gal-derived culture supernatant were added to the substrate after gelatinization treatment. The viscosity value of the slurry after saccharification was 18.0 mPa·s.

### <Test Example 23>

The enzyme units in the culture supernatants derived from SP21 and SP21·Gal were measured. As a result, the enzyme units of these enzymes were as shown in Table 9.

**[Table 9]**

| Enzyme activity (U/mg-total protein) | Cellulase activity | Pga activity | Pel activity | Abf activity | Gal activity | Pectinase content per protein weight |
|---|---|---|---|---|---|---|
| SP21 | 2.9 | 45.1 | 3.6 | 0 | 0.7 | 8.9% |
| SP21·Gal | 3.2 | 49.0 | 3.6 | 0 | 20.9 | 13.1% |

## Claims

1. An enzyme composition comprising the following (A), (B), and (C):
(A) cellulase;
(B) a homogalacturonan-degrading enzyme containing polygalacturonase and pectin lyase; and
(C) one or more members selected from the group consisting of arabinofuranosidase and galactanase.

2. The enzyme composition according to claim 1, for saccharification of plant biomass.

3. The enzyme composition according to claim 2, wherein a viscosity of the saccharified plant biomass is reduced compared to a case of saccharification using cellulase alone.

4. The enzyme composition according to any one of claims 1 to 3, which has a cellulase activity of 2.0 to 3.5 U per mg of protein in the enzyme composition.

5. The enzyme composition according to any one of claims 1 to 4, which has a polygalacturonase activity of 30 U to 1200 per mg of protein in the enzyme composition.

6. The enzyme composition according to any one of claims 1 to 5, which has a pectin lyase activity of 1 to 27 U per mg of protein in the enzyme composition.

7. The enzyme composition according to any one of claims 1 to 6, which has an arabinofuranosidase activity of 0.1 to 50 U per mg of protein in the enzyme composition.

8. The enzyme composition according to any one of claims 1 to 7, which has a galactanase activity of 3 to 250 U per mg of protein in the enzyme composition.

9. The enzyme composition according to any one of claims 1 to 8, wherein the ratio of the homogalacturonan-degrading enzyme (B) and one or more members selected from the group consisting of arabinofuranosidase and galactanase (C) ((B)+(C)) contained in protein in the enzyme composition is from 1 mass% to 50 mass%.

10. A method for saccharifying plant biomass, comprising bringing the enzyme composition according to any one of claims 1 to 9 into contact with plant biomass.

11. A method for producing sugar from plant biomass, comprising bringing the enzyme composition according to any one of claims 1 to 9 into contact with plant biomass.

12. A method for producing ethanol from plant biomass, comprising bringing the enzyme composition according to any one of claims 1 to 9 into contact with plant biomass.

13. The method according to any one of claims 10 to 12, wherein the plant biomass is pectin-containing plant biomass.

14. The method according to any one of claims 10 to 12, wherein the plant biomass is cassava or a residue thereof.

15. The method according to claim 13 or 14, wherein a viscosity of the plant biomass which has been brought into contact with the enzyme composition according to any one of claims 1 to 9, compared to a case of contact using cellulase alone.

16. A recombinant filamentous fungus comprising (b1) a polygalacturonase gene, (b2) a pectin lyase gene, and (c) one or more genes selected from the group consisting of arabinofuranosidase gene and galactanase gene which are introduced.

17. A method for producing the enzyme composition according to any one of claims 1 to 9, comprising culturing the recombinant filamentous fungus according to claim 16.

18. The recombinant filamentous fungus according to claim 16, wherein the polygalacturonase gene (b1) is PgaB gene, the pectin lyase gene (b2) is PelD gene, and (c) is Abf gene or Gal gene.

19. The method according to claim 17, wherein the polygalacturonase gene (b1) is PgaB gene, the pectin lyase gene (b2) is PelD gene, and (c) is Abf gene or Gal gene.

20. An agent for reducing a viscosity of plant biomass comprising the enzyme composition according to any one of claims 1, 2, and 4 to 9 as an active ingredient.

21. A method for reducing a viscosity of plant biomass, comprising bringing the enzyme composition according to any one of claims 1, 2, and 4 to 9 into contact with plant biomass.

22. Use of the enzyme composition according to any one of claims 1, 2, and 4 to 9 for reducing a viscosity of plant biomass.
